# EUROPEAN PATENT SPECIFICATION

(11) **EP 4 536 302 B1**
(45) Date of publication and mention of the grant of the patent: **25.11.2026**
(21) Application number: 23731675.7
(22) Date of filing: 08.06.2023
(51) Int. Cl.: A61L 27/26, A61L 27/52

(54) **INJECTABLE HYDROGEL**
INJIZIERBARES HYDROGEL
HYDROGELS INJECTABLES

(30) Priority: 08.06.2022 EP 22177724
(43) Date of publication of application: 16.04.2025
(73) Proprietor: Allegro NV, 2020 Antwerp (BE)
(72) Inventor: DECUYPERE, Lucas, 2020 Antwerp (BE); SCHELFHOUT, Jorg, 2020 Antwerp (BE)
(74) Representative: Cooley (UK) LLP
(86) International application number: PCT/EP2023/065423
(87) International publication number: WO 2023/237704

(56) References cited:
- WO-A1-2022/023815
- CN-A- 110 760 103
- US-A1- 2015 297 371
- BALAKRISHNAN BIJI ET AL: "Borate aided Schiff's base formation yields in situ gelling hydrogels for cartilage regeneration", JOURNAL OF MATERIALS CHEMISTRY. B, vol. 1, no. 41, 1 January 2013 (2013-01-01), GB, pages 5564, XP093001236, ISSN: 2050-750X, DOI: 10.1039/c3tb21056a
- RESMI RAJALEKSHMI ET AL: "Injectable self-crosslinking hydrogels for meniscal repair: A study with oxidized alginate and gelatin", CARBOHYDRATE POLYMERS, APPLIED SCIENCE PUBLISHERS , LTD BARKING, GB, vol. 234, 26 January 2020 (2020-01-26), XP086051184, ISSN: 0144-8617, [retrieved on 20200126], DOI: 10.1016/J.CARBPOL.2020.115902

## Description

The present description relates to an injectable hydrogel, a method to produce the injectable hydrogel and the use of the injectable hydrogel to treat a pathological joint, for example having a disease such as arthritis.

Joints or articulations are essential to the mobility of humans and animals. They are filled with synovial fluid and include articular cartilage as a complex, nonlinear, viscoelastic, and anisotropic material that is subjected to millions of cycles of joint loading over decades of wear. Pathological joints may lead to important pain and reduced mobility and thus an important degradation of the life quality of the affected animals or human beings. For example, pathological joints may have different injuries or diseases such as arthritis, for example rheumatoid arthritis, osteoarthritis or other form of arthritis, but also cartilage degeneration, cartilage injuries or subchondral defects, resulting in reduced mobility and a chronic pain.

3D printing has been developed recently in order to obtain bespoke scaffolds to be implanted in impaired joints, for example due to an accident or a trauma. Nevertheless, the impaired joint must be opened in order to implant the printed scaffold into it, which requires a heavy surgical procedure and which may lead to a long recovery time for patients, with the risk that the implant may be rejected by the body or lead to deceptive results, such a limited mobility improvement or limited pain relief. In addition, printed scaffolds are not adapted to cure joint diseases such as osteoarthritis.

Arthritis such as Osteoarthritis (OA) is a musculoskeletal condition and the largest cause of disability in the world. Although OA is often referred to as a joint disease with damage and loss of cartilage, OA is a much more diverse disease with complex pathogenesis that affects all tissues within the joint. The primary characteristic of OA includes the progressive loss of the articular cartilage tissue, synovial tissue inflammation, disrupted characteristics of the synovial fluid, subchondral bone sclerosis and osteophyte formation at the margin of the joint, which results in chronic pain, joint stiffness and eventually impaired mobility.

Hydrogels are known in the art. For example, CN 110760103 relates to a viscoelastic hydrogel as well as preparation method and application thereof. Balakrishnan et al., Journal of Materials Chemistry B 2013, 1, 5564, discloses borate aided Schiff's base formation yields *in situ* gelling hydrogels. If these hydrogels are disclosed for cartilage regeneration, they are not suitable for relieving the pain and increasing mobility of a joint having arthritis.

The present invention relates to an injectable hydrogel including:
- oxidized nanocrystalline cellulose,
- gelatine peptide chains,

wherein the oxidized nanocrystalline cellulose and the gelatine peptide chains are cross-linked with a cross-linking degree of 25 to 60%, and
wherein the injectable hydrogel has a concentration of 1% to 4% w/v.

Such a hydrogel may be injected in a pathological joint, for example having arthritis such as type-I OA, to restore the viscoelastic properties of the joint and to relieve pain, due to the very high miscibility of the hydrogel in synovial fluid and its favourable viscoelastic properties. Alternatively, the present injectable hydrogel may be injected in other parts of a mammal such as in an impaired eye. The gelatine peptide chains may not be functionalized, i.e. may include naturally occurring moieties only. The oxidized nanocrystalline cellulose may include aldehyde moieties or may have reactive moieties consisting essentially of aldehyde moieties. The hydrogel may then be prepared under the form of stable colloids of gel particles in water buffered at physiological pH.

Advantageously, the oxidized nanocrystalline cellulose and the gelatine peptide chains are cross-linked by imine moieties. The imine or aldimine moieties may result preferably from the direct reaction of the oxidized nanocrystalline cellulose with unmodified gelatine peptide chain, for example by the Schiff's base reaction. Consequently, no bridge or linker may be used to obtain the present cross-linked hydrogel.

Advantageously, the injectable hydrogel includes a buffer to maintain the pH of the injectable hydrogel from 7 to 8, preferably from 7.2 to 7.6. For example, the buffer is Dulbecco's Phosphate-Buffered Saline (DPBS) but other buffer close or within the desired pH ranges may be considered. The buffer allows to increase biocompatibility of the injectable hydrogel when injected in a joint. The buffer may also contribute to an efficient cross-linking reaction.

Advantageously, the concentration in the composition before cross-linking of the gelatine peptide chains is 1.0 to 2.5 weight/volume %, preferably 1.5 to 2.1 weight/volume %, and at most preferably 1.7 to 1.9 weight/volume %. Alternatively or in combination, the oxidized nanocrystalline cellulose has a concentration before cross-linking of 0.1 to 0.5 weight/volume %, preferably 0.2 to 0.4 weight/volume %, and at most preferably 0.3 weight/volume %. A composition leading to an efficient cross-linking and the present hydrogel may be obtained from a combination of any of the above concentrations of oxidized nanocrystalline cellulose and gelatine peptide chains.

Advantageously, the injectable hydrogel includes 0.5 to 2.5 weight/volume % of cross-linked oxidized nanocrystalline cellulose and gelatine (i.e. gel fraction), preferably 0.8 to 2 w/v%, for example 1.5 or 1.9 w/v%. The injectable hydrogel has a concentration of 1 to 4 (w/v)%*.* These concentrations of hydrogel may allow an easy and painless injection into a pathological joint while efficiently restoring mobility.

Advantageously, the injectable hydrogel has an elastic modulus of 10.0 to 220.0 Pa from 0.1 to 10.0 Hz, preferably 50.0 to 200.0 Pa, and most preferably at most 80.0 Pa, for example 120.0 Pa. Such an elastic modulus allows for an optimal treatment of a pathological joint.

Advantageously, the injectable hydrogel has a loss modulus below 10.0 Pa from 0.1 to 10.0 Hz, preferably below 9.0 Pa. Such a loss modulus allows for an optimal treatment of a pathological joint allowing restoring a high level of mobility. For example, the loss modulus may be at least 5.0 Pa, at least 6.0 Pa or at least 7.0 Pa from 0.1 to 10.0 Hz.

Advantageously, the injectable hydrogel has a cross-linking degree of 38 to 51%, at most preferably 42 to 49%. A higher cross-linking degree may lead to a hydrogel difficult to inject and may have a negative impact on a pathological joint. A lower cross-linking rate may not cure or relieve a pathological joint.

Preferably, the injectable hydrogel is sterilized and/or homogeneous, without any aggregate, insoluble particle or solid phase. Further, the injectable hydrogel may be fully homogeneous and/or isotropic when mixed with synovial fluid, i.e. form a stable colloid without solid or supernatant in order to limit inflammation and restore mobility of an impaired joint. The hydrogel may be directly injected once crosslinked.

Another aspect of the present invention is a method to prepare an injectable hydrogel according to any of the previous claims, the method including the steps of:
- Diluting oxidized nanocrystalline cellulose within apyrogenic water
- Adding gelatine peptide chains to the diluted oxidized nanocrystalline cellulose at room temperature
- Reacting the gelatine peptide chains and the oxidized nanocrystalline cellulose.

The above method may allow to obtain the present hydrogel for a limited cost. If gelatine peptide chains are added at low temperature, gelatine peptide chains may have a helix confirmation leading to a non-homogeneous hydrogel and preventing a safe injection.

Advantageously, the reacting step is performed for 10 to 25 hours and/or at room temperature, for example 15 to 25 °C. A higher temperature cross-linking reaction may prevent an efficient cross-linking reaction. A low temperature cross-linking reaction may lead to a non-homogeneous hydrogel.

Advantageously, the reaction step may be performed at a mildly alkaline pH, for example more than 6 to at most7.5 and preferably 7.2 to 7.6. A pH buffer may be used, for example DPBS buffer, for example with no stirring.

Advantageously, gelatine peptide chains are prepared by a dilution step in a pyrogenic water at 37 °C and the diluted gelatine peptide chains are then cooled to room temperature, such as 20 °C or at least between 15 and 25 °C. This allows for an optimal dilution of the gelatine peptide chains and prepare the gelatine peptide chains for addition to the nanocrystalline cellulose.

Another aspect of the present invention is a medical syringe filled with the above injectable hydrogel. Such a medical syringe is advantageous for a quick injection into a pathological joint, for example having OA. The medical syringe may be pre-filled, i.e. filled up in a factory before delivery to patients and medical staff.

Preferably, the medical syringe includes a single barrel defining a reservoir containing the hydrogel (i.e. after the cross-linking reaction) and a stopper to close an extremity of the barrel, the stopper being movable within the barrel. The medical syringe may preferably include a needle and/or a needle adapter for a direct injection of the injectable hydrogel into a pathological joint. The needle may range from 27 gauge to 17 gauge, preferably 27 to 19 gauge, for example 25 to 21 gauge. The hydrogel may be stored in the syringe and/or injected with the syringe under the form of a stable colloid in water buffered to physiological pH, for example pH = 7.2 to 7.4.

Another aspect of the present invention is the above injectable hydrogel for use in relieving the symptoms of osteoarthritis, for example in a mammal such as a horse or a human being.

Another aspect of the present disclosure is a hydrogel for use in a method to treat or cure a pathological joint, in particular a joint having OA, the method including a step of providing an injectable hydrogel as disclosed above and a step of injecting such injectable hydrogel into the pathological joint.

Further advantages and preferred embodiments of the present disclosure will become apparent from the following detailed description and drawings, in which:
Figure 1 shows the elastic modulus (G') and viscous modulus (G") as functions of frequency for the synovial fluid of a normal middle intercarpal joint and a pathological middle intercarpal joint, extracted from horses.
Figure 2 shows a schematic reaction to prepare nanocrystalline cellulose.
Figure 3 shows several possible saccharide units of nanocrystalline cellulose after an oxidation reaction.
Figure 4 shows a developed formula of typical amino acids in a gelatine peptide chain.
Figure 5 shows elastic (G') and viscous (G") moduli as functions of frequency for a 2% (w/v) hydrogel according to the disclosure.
Figure 6 shows the loss factor (tan δ) and the complex viscosity of the 2% (w/v) hydrogel.
Figure 7 shows elastic (G') and viscous (G") moduli as functions of frequency, for pathological synovial fluid (OA SF) extracted from intercarpal an joint and for the same pathological synovial fluid mixed with 10% volume/volume of hydrogel 2.0% (w/v).
Figure 8 shows elastic (G') and viscous (G") moduli as functions of frequency, of pathological synovial fluid mixed with 10% volume/volume of hydrogel 2.0% (w/v) (OA SF +10% hydrogel) and of a normal synovial fluid (OA SF) extracted from equine intercarpal joint.
Figure 9 shows the shear stress of a pathological synovial fluid extracted from an osteoarthritic joint (intercarpal joint - OA SF) in comparison with the same fluid mixed with 10% (v/v) hydrogel 2% (w/v) (OA SF + 10% hydrogel 2%).
Figure 10 shows the shear stress of a pathological synovial fluid extracted from another osteoarthritic joint (equine tarsus) in comparison with the same fluid mixed with 10% (v/v) hydrogel 2% (w/v) (OA SF + 10% hydrogel).
Figure 11 shows the cross-linking reaction monitored by the evolution of elastic modulus G' overtime, for a composition according to the present disclosure (Example) and a composition including non-oxidized nanocrystalline cellulose (Comparative Example 1).
Figure 12 shows the cross-linking reaction monitored by the evolution of elastic modulus G' overtime for the same composition at 20 °C (Example) and at 40 °C (Comparative Example 2).
Figure 13 shows the impact of the pH of the cross-linking reaction of the elastic modulus G' of corresponding hydrogel.
Figure 14 shows the in-vivo stability of a 2% w/v hydrogel under physiological conditions overtime.

The present disclosure includes a hydrogel adapted to be injected into a joint in order to increase mobility and relieve joint pain, for example linked with osteoarthritis. The present inventors studied the alterations in the viscoelastic properties of osteoarthritic equine synovial fluid (SF) and determine that in osteoarthritic joints, the rheological properties of the synovial fluid are degraded. In particular, the synovial fluid becomes less viscous, less elastic and consequently less effective in joint lubrication.

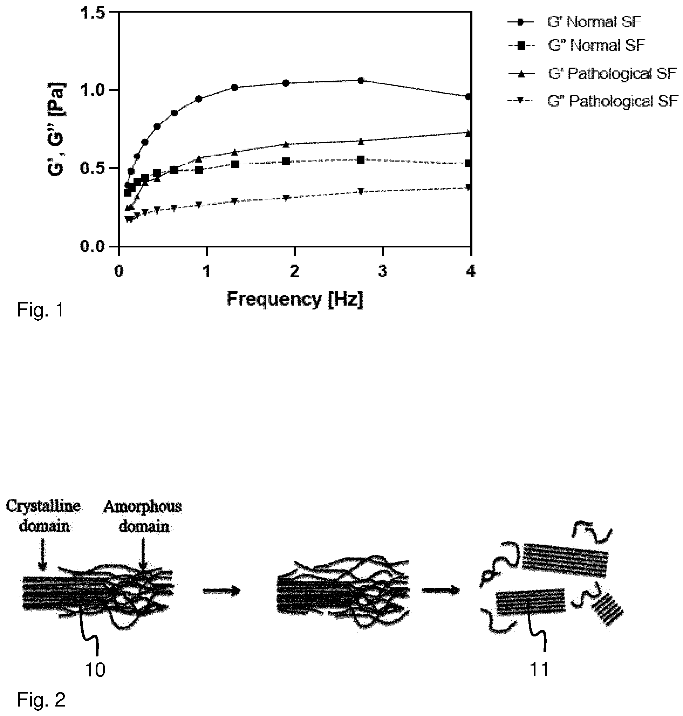

In Fig. 1, measurements of elastic modulus (G') and viscous modulus (G") as functions of frequency are shown for equine synovial fluid of a normal middle intercarpal joint and a pathological middle intercarpal joint (having osteoarthritis type I). It can be observed that in orthopedically normal SF and in pathological SF, the elastic component (G') is prevalent over the viscous component (G") for all the frequency deformations. However, the absolute values of G' and G" of the normal SF are significantly higher in comparison with pathological SF.

The hydrogel according to the present disclosure may restore joint lubrication when injected in an osteoarthritic joint, thus improving mobility and relieving joint pain of the patient or the animal.

The hydrogel may include at least two components: cellulose and gelatine peptide chains, in addition to purified water. However, other components may be added such as a pH buffer, growth factors, proteins, hyaluronic acid and/or polycaprolactone. Alternatively or in combination, another type of polysaccharide may be used to form an hydrogel with gelatine peptide chains, for example chitosan.

Cellulose may be extracted from a biomass source or from bacteria. Cellulose is preferably under the form of nanocrystalline cellulose or cellulose nanocrystals, for example excluding cellulose nanofibers. The cellulose nanocrystals may have a bar shape comprising distinct faces, such as six faces and dimensions of 100 to 900 nm in length, preferably 150 to 850 nm, again preferably 240 to 760 nm, 370 to 620 nm, 410 to 580 nm and at most preferably around 500 nm. The width and height of the crystals may be 5 to 40 nm, preferably 9 to 32 nm, again preferably 10 to 25 nm, and most preferably 15 to 20 nm.

Nanocrystalline cellulose may be obtained by hydrolysis according to Reaction 1 below and Fig. 2.

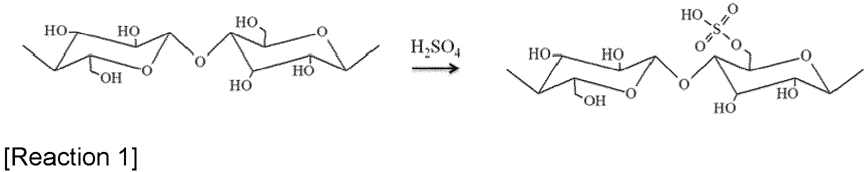

In Fig. 2, cellulose 10, for example extracted from a biomass source such as cotton or from a bacteria source, may comprise crystalline and amorphous domains and the amorphous domains may be cut by the acidic hydrolysis, for example using sulfuric acid in order to obtain only the crystalline domains, preferably under the form of nanocrystals 11. Depending on the reaction conditions, different shapes of nanocrystals may be obtained while the bar shape may provide favourable rheological properties to the composition. In contrast with its low molecular weight homologues, nanocrystalline cellulose may have a limited diffusivity due to their macromolecular structure, which is advantageous for intra-articular injection.

Nanocrystalline cellulose may have terminal aldehyde reactive moieties. In addition, nanocrystalline cellulose may be functionalized to increase the number of reactive moieties or add additional reactive moieties. Consequently, the number or type of the additional reactive moieties may differ from the reactive moieties of natural and/or nanocrystalline cellulose. Preferably, the reactive moieties include C=O double bonds and are preferably aldehyde moieties. These aldehyde moieties may be obtained through oxidation of nanocrystalline cellulose, for example by a strong oxidant such as periodate, as illustrated in the below Reaction 2.

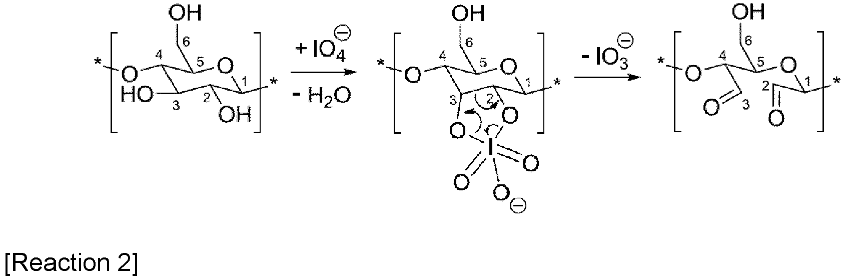

According to Reaction 2, a periodate ion may be linked to two hydroxyl moieties of a saccharide cycle of the cellulose, by elimination of a water molecule. Electron rearrangement leads to opening the saccharide cycle, thus providing a di-aldehyde chain instead of the saccharide cycle. The reactive moieties are thus preferably aldehyde moieties, for example mainly or only aldehyde moieties.

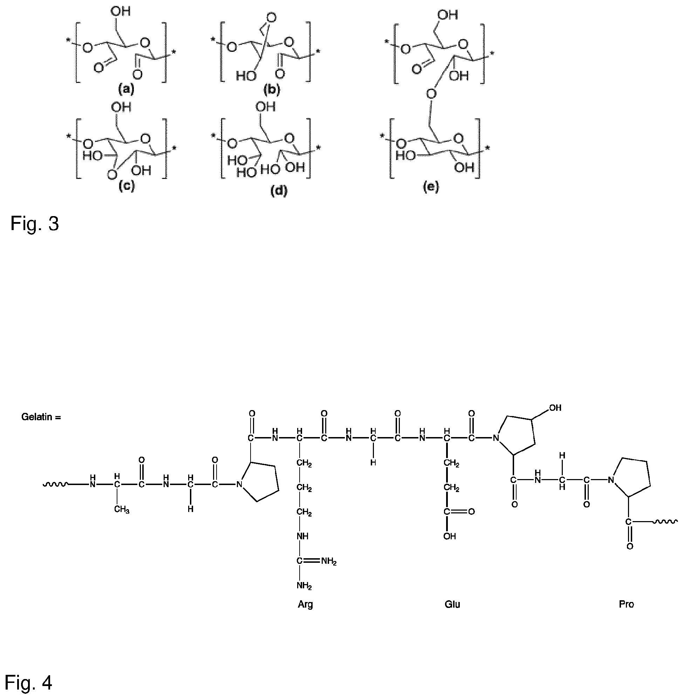

Oxidation at positions C₂ and C₃ may thus lead to the cleavage of the saccharide cycle, which alters the structure of the polysaccharide chain. An increase in the backbone flexibility of the polymer may be obtained. Sterically stabilized nanocrystalline cellulose can be obtained by the oxidation step which introduces flexible units in the polysaccharide chains. The flexible units may enable or contribute to colloidal stability of the hydrogel. Here, the di-aldehyde units formed by the oxidation reaction may be in equilibrium with other units, as visible in Fig. 3.

Fig. 3 represents different cycles or units of cellulose that may be formed after the above oxidation reaction. Fig 3(a) shows free aldehyde moieties, Fig. 3(b) shows intramolecular hemiacetal moiety, Fig. 3(c) shows a hemialdol moiety, Fig. 3(d) shows hydrated aldehydes, i.e., hydroxyl moieties produced by hydration of the aldehyde moieties and Fig. 3(e) shows intermolecular hemiacetal. While the intermolecular hemiacetal of Fig. 3(e) may contribute to cross-linking between chains of nanocrystalline cellulose, these hemiacetal bridges are rare and mostly occurs between chains of a same cellulose nanocrystal.

The aldehyde content may be assessed through a UV/vis spectrophotometry (BCA assay) with D-(+)-glucose as a standard. For example, the aldehyde content is 800 to 3500 µmol/g of oxidized nanocrystalline cellulose, preferably 1000 to 2800 µmol/g, again preferably 1400 to 2200 µmol/g and most preferably around 1900 µmol/g.

Gelatine may be any gelatine compatible with human or with the animal species intended to be treated. In particular, type-B gelatine may be used. The gelatine is preferably non-functionalized and may only include naturally occurring reactive moieties, as visible in Fig. 4.

Gelatine may include gelatine peptide chains, preferably obtained from bovine gelatine because the iso-electric point of bovine gelatine is close to the iso-electric point of the human body. However, other types of gelatine may be used, in particular for non-human applications. Fig. 4 shows a (partial) structure of gelatine, for example showing Arginine, Glycine and Proline. However, other natural amino acids may be present in gelatine, as known by the skilled person.

The present hydrogel may be cross linked by reaction between the oxidized nanocrystalline cellulose and the gelatine peptide chains, in particular by reaction between nucleophile moieties of the gelatine and the aldehyde moieties of the oxidized nanocrystalline cellulose. For example, cross-linkage may be achieved through Schiff's base reaction, according to Reaction 3.

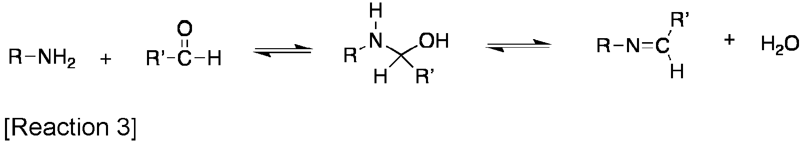

According to Reaction 3, the ε-amine moieties of lysine and hydroxylysine amino acids of gelatine may react with the available aldehyde moieties of the oxidized nanocrystalline cellulose, thus leading to imine moieties linking the peptide chains and the saccharide chains of gelatine and nanocrystalline cellulose, receptively. This reaction is preferably conducted at room temperature. Indeed, room temperature allows to efficiently obtain a pure hydrogel, ready for injection. In contrast, the inventors found out that the use of a higher temperature may prevent an efficient cross-linking reaction.

For example, the molar ratio of gelatine on oxidized nanocrystalline cellulose may be of 1.8 to 0.2, preferably 1.5 to 0.5, again preferably 0.8 to 1.2, most preferably 1. The cross-linking degree may be defined as the ratio of the amount of substance of imine moieties in the hydrogel to the amount of substance of aldehyde moieties on the oxidized cellulose before forming the hydrogel. The cross-linking degree may be at most 70% or at most 60%, preferably 38 to 55%, again preferably 45 to 53% and for example approximately 49%. Such a cross-linking degree allows for a good injectability.

The present hydrogel may be rheopectic. For example, the viscosity of the hydrogel may be 100-500 Pa.s at a shear rate of 0.1 s⁻¹ and after 100 s.

### Rheological Analysis

The investigation of the rheological behaviour was carried out on an Anton Paar Modular Compact Rheometer MCR 302e. A plate-plate geometry was used (PP25) with a 0.5 mm gap. The properties of the samples were examined at the physiological horse's temperature, i.e. at 37.5 °C, with an accuracy of 0.1 °C provided by the instrument's Peltier plate temperature control system.

### Oscillation Tests and Viscoelasticity

The viscoelastic properties of the samples were determined via dynamic experiments. The storage modulus (or elastic modulus) (G'), the loss modulus (or viscous modulus) (G"), the loss tangent (tan δ), and complex viscosity (η*) were monitored as functions of frequency.

The loss tangent (tan δ) is defined according to Equation 1. [Equation 1] $tan δ = \frac{G "}{G ′}$

The complex viscosity η* is a measure of the total resistance to flow as a function of the angular frequency (ω) and is given by the quotient of the maximum stress amplitude and maximum strain rate amplitude.

A dynamic strain scan test was also performed as follows: at a fixed frequency, a strain scan as applied from 0.1 to 100% to determine the linear viscoelastic range. A dynamic frequency scanning test: the strain constant was maintained at 10% and a frequency scan from 0.1 to 4 Hz was applied. The frequency range studied encompassed the physiological frequencies of knee movements (0.5 Hz for slower knee movements, rising to 3 Hz).

### Rotational Tests and Viscosity

Rotational and viscosity test were performed at a constant shear rate of 0.01 s⁻¹ on the synovial fluid samples. The test immediately followed a pre-shear at 100 s⁻¹ for 60 sec and t=0 is shortly after the end of the pre-shear and the start of the application of 0.01 s⁻¹ during 600 sec. The shear stress τ is according to Equation 2, wherein F is the shear force (in N) and A the shear area A (in m²). [Equation 2] $τ = \frac{F}{A}$

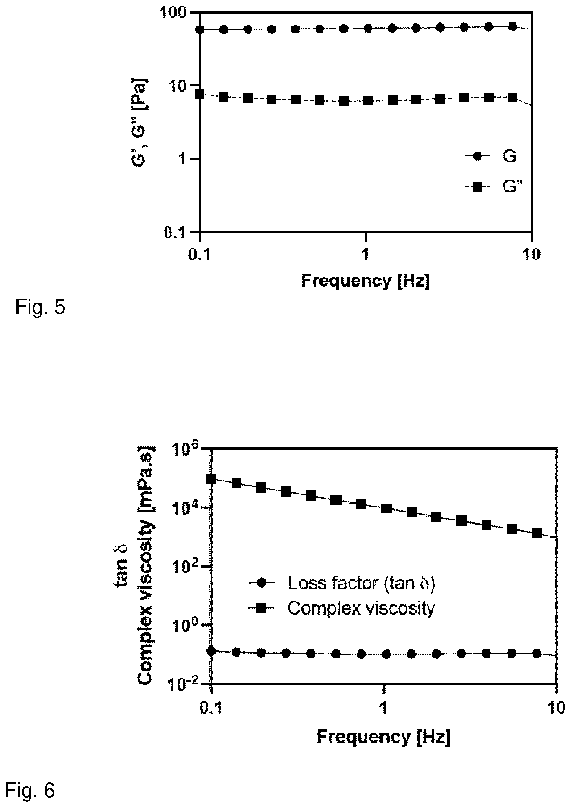

The viscoelastic properties (loss G" and elastic G' moduli) as a function of frequency for an injectable hydrogel of 2.0% w/v are shown in Figure 5, while complex viscosity and tan δ dependence on frequency are reported in Figure 6. The rheological behaviour of the present hydrogel is typical of a weak gel. Throughout the frequency range, the elastic modulus (G') is always higher than the viscous modulus (G") by about one order of magnitude, and both moduli are frequency independent.

Further the elastic modulus G' is always above 10 Pa from 0.1 to 10.0 Hz, preferably above 50 Pa and again preferably above 80 Pa. The loss modulus G" is always below 10 Pa, preferably below 9 Pa in the same frequency range.

### In Vitro Results

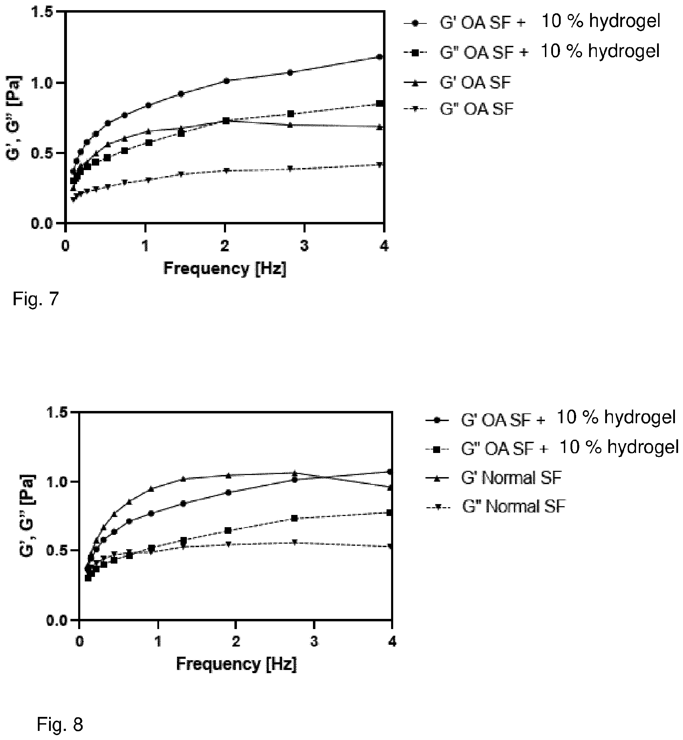

To demonstrate the curing properties of the present hydrogel for arthritic joints, the present hydrogel at a concentration of 2% (w/v) in PBBS buffer has been studied in mixture with horse synovial fluid (10% volume of hydrogel in pathological synovial fluid, i.e. 1 ml of hydrogel in 9 ml of synovial fluid), in order to mimic what occurs in vivo after injection. Fig. 7 shows elastic (G') and viscous (G") moduli as functions of frequency, from pathological synovial fluid (OA SF) and pathological synovial fluid mixed with 10% of the above hydrogel (v/v), i.e. 1 ml of hydrogel in 9 ml of pathological synovial fluid. As seen in Fig. 7, the viscoelastic modulus values of the pathological synovial fluid (OA SF) increase for all the deformation frequencies when mixed with the present hydrogel.

Fig. 8 shows elastic (G') and viscous (G") moduli as functions of frequency, of pathological synovial fluid mixed with 10% hydrogel 2.0% (w/v) and a normal synovial fluid (normal SF), both extracted from an equine intercarpal joint. This experiment allows to mimic a pathological joint cured by the present hydrogel with regard to a normal joint. The values of elastic (G') and viscous (G") moduli of the pathological synovial fluid mixed with the present hydrogel is close or similar to the values obtained by the synovial fluid of a normal, healthy joint. These results allow to conclude that the present hydrogel may be successfully injected into a pathological joint having osteoarthritis in order to relieve pain and restore mobility, at least partially.

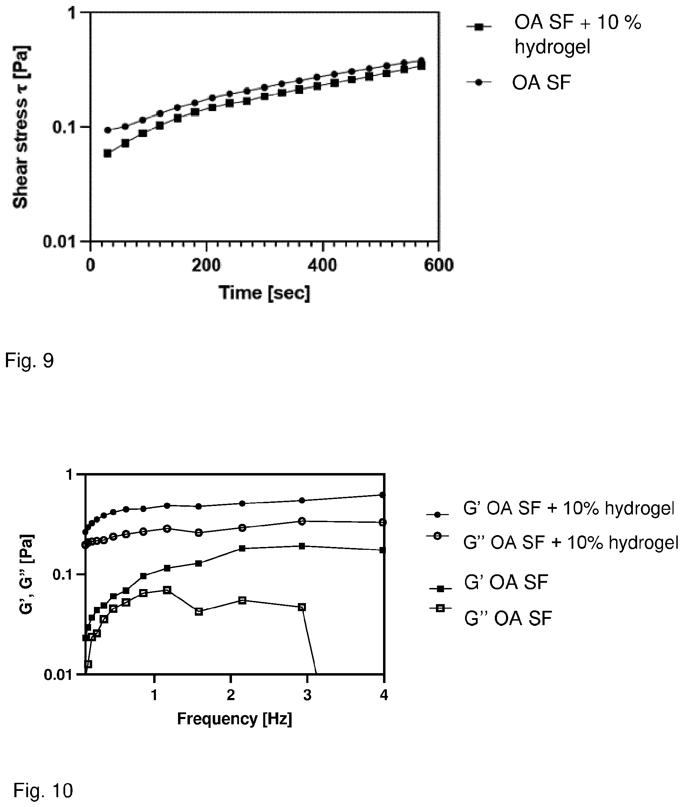

The shear stress τ as a function of time is shown in Figure 9 for pathological synovial fluid and pathological synovial fluid mixed with 10% (v/v) of the present hydrogel at 2.0%(w/v). Figure 9 shows that both samples exhibit veery close shear stress values and a rheopectic behaviour, defined as a shear stress increasing over time at a constant shear rate.

Fig. 10 results to the same experiment conducted in Fig. 7 but using synovial fluid of a different joint: equine tarsus joint. Both moduli of the pathological synovial fluid are low, while the loss modulus G" decreases quickly beyond 1 Hz (i.e. fast walk and running). In contrast, when mixed with the present 10% (v/v) hydrogel 2% (w/v), both moduli are increased. At low frequencies (i.e. walk) the mixed synovial fluid maintains its viscous character, while at higher frequencies (running), the mixed synovial fluid tends to maintain its elastic properties, potentially protecting the articular cartilage from unmitigated forces.

Then, detailed examples of procedures that may be used to obtain the present hydrogel are provided as follows. These procedures do not limit the above general disclosure but are provided to enable sufficiency of disclosure.

### Preparation of the Oxidized Nanocrystalline Cellulose

In a two-neck flask of 1L, 300 ml demineralized water is added and acidified with 5.5 ml acetic acid (36%) to a pH of 3.0. Under mechanical stirring, 6.0 g of cellulose nanocrystals is suspended. 92.5 ml of a 0,7M NaIO₄ solution is added dropwise to the mixture. The flask is protected from light, and the mixture is allowed to react for 4 hours at 45 °C.

Ethylene glycol is added (5.0 ml) to stop reaction and the pH is adjusted to 6.0 with Na₂CO₃ (1M, 17.3 ml) and stirred during an extra hour. The final mixture is dialyzed (SpecPor 5, 12-14 kDa) against water (28L) at room temperature for 5 days with 3 times a renewal of the water until a total dissolved solids content (TDS) of 0 ppm is obtained.

### 2.0% (w/v) Hydrogel Preparation Example

3.2 ml of the dialdehyde nanocrystalline cellulose (aldehyde content: 1891 µmol/g) suspension is diluted in apyrogenic water to a total volume of 10 ml. 170 mg of gelatine type B (primary amine content: 435 µmol/g) is diluted in apyrogenic water at a temperature of 37 °C and then cooled down to 20°. The diluted gelatine is added to the dialdehyde nanocrystalline cellulose at a temperature of 20 °C with 96 mg of Dulbecco's Phosphate-Buffered Saline (DPBS) to buffer the solution to a pH of 7.2-7.4. No stirring was performed during cross-linking.

The cross-linking reaction is completed after 20 h at room temperature whereby a visco-elastic hydrogel is obtained. The hydrogel may be maintained at a temperature of 15-25 °C during storage and then sterilized with gamma irradiation such as a C⁶⁰ gamma source of 30 kGy, i.e. at least 25 kGy. The efficacy of the cross-linking reaction (Schiff base reaction) is assessed by a TNBS assay, whereby the concentration of the primary amines is determined. Alternatively, an acid-base titration with hydroxylamine was also performed. Based on this data, we can conclude that all the aldehydes are quantitatively reacted with the amines and resulted in a cross-linking degree of the gelatine of 49.9%.

### BCA Method, Determination of Aldehyde Groups in Oxidized Nanocrystalline Cellulose

The oxidized nanocrystalline cellulose sample is diluted to 0.01% concentration with BCA buffer (1.0 M KCl; 0.01M Na₂CO₃; 0.09 M NaHCO₃). For the calibration curve, 0.09008 g of D-(+)-glucose is dissolved with BCA buffer in a 500 ml volumetric flask resulting in a 1000 µM D-(+)-glucose stock solution. A standard series was prepared by diluting the stock solution with the following concentrations: 0, 50, 100, 250, 500 µM. The standard was treated such as the diluted sample. 100 µL of the diluted sample/standard is transferred to a glass vail with a cap and 900 µL of BCA buffer is added. Then, 1000 µL of freshly prepared BCA reagent solution (0.2561M Na₂CO₃; 0.1440M NaHCO₃; 0.0025M BCA; 0.0039M CuSO₄•5H2O; 0.0060M) is added. The glass vials were then heated in a water bath for 30 min. At 75 °C. The absorbance was measured in a double beam spectrophotometer at 560 nm wavelength against demineralized H₂O.

The aldehyde content is preferably of 1500 to 2500 µmol/g of nanocrystalline cellulose, preferably 1700 to 2300 µmol/g, again preferably 1800 to 1900 µmol/g.

### TNBS Method, determination of free primary amino groups in cross-linked hydrogel samples

10 µL of the sample was transferred to an amber glass vial with a screw cap and 990 µL of TNBS buffer were added (0.1M NaHCO₃; pH 8.5), the gelatine concentration of the diluted sample was approximately 0.01% (w/v). To draw a calibration curve, a stock solution was prepared by weighing 0.0563 g of L-glycine with an analytical balance and dissolved with TNBS buffer in a 500 ml volumetric flask to a final concentration of 1500 µM of L-Glycine. The stock solution was then diluted with TNBS buffer to prepare a standard series with the following concentrations: 10, 25, 50, 75, 100, 150 µM. For each standard, 1000 µL was transferred to an amber glass vial with a screw cap and treated in the same way as the sample.

The amine content is preferably from 220 to 840 µmol/g of gelatine peptide chains, preferably 350 to 520 µmol/g and at most preferably 390 to 450 µmol/g.

A 0.05% TNBS solution was freshly prepared by diluting 5% TNBS with the TNBS buffer. 500 µL of the freshly prepared 0.05% TNBS solution was added to each vial. The solutions were incubated in an oven for 2 hours at 37 °C. After the incubation, 250 µL of 1M HCL and 500 µL of demineralized H₂O was added to each vial to stop the reaction. All the solutions were then hydrolysed in an autoclave at 120 °C and approximately 15 psi (103421 Pa) for 1 hour. After the hydrolysis, approximately 2 ml of the solutions were transferred to UV cuvettes with a path length of 1 cm and measured in a double beam spectrophotometer at 335 nm wavelength against a blank that was prepared analogue to the sample.

### Cross-Linking Reaction

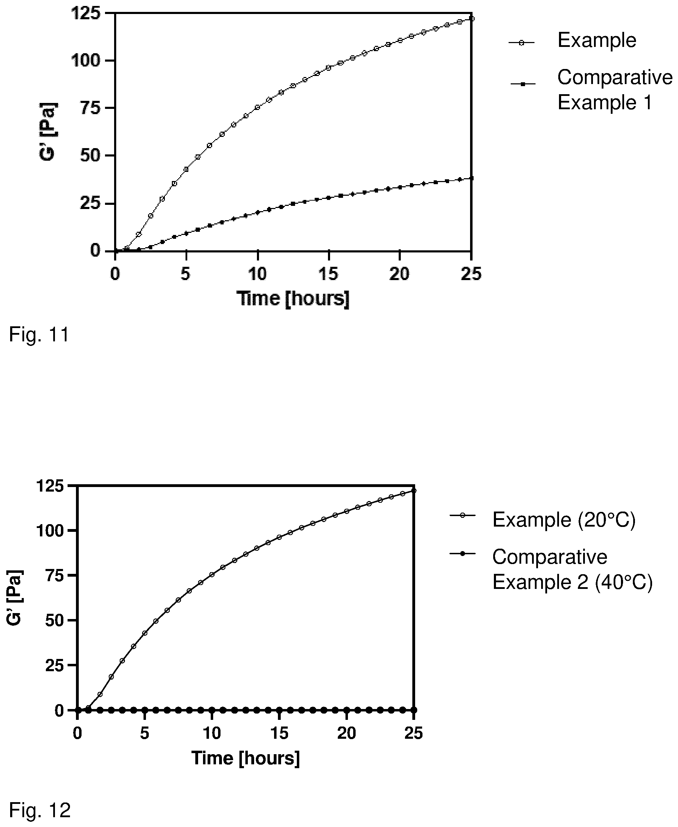

To investigate the efficiency of the cross-linking reaction, a first composition has been prepared including the above gelatine peptide chains and oxidized nanocrystalline cellulose (Example in Fig. 11) and a second composition has been prepared including the above gelatine peptide chains and non-oxidized nanocrystalline cellulose, i.e. including only naturally occurring aldehyde moieties (Comparative Example 1 in Fig. 11). Both compositions have been incubated at 20 °C without stirring and the elastic modulus of both compositions has been investigated overtime (i.e. up to 25 hours).

As visible in Fig. 11, elastic modulus G' for the Comparative Example 1 increases slightly overtime, indicating a physical gel formation. In contrast, elastic modulus G' for the Example (i.e. the present hydrogel 2% w/v) increases strongly overtime, indicating a physical gel formation and an efficient cross-linking. Using oxidized nanocrystalline cellulose thus allows to obtain appropriate rheological and mechanical properties of the present hydrogel by an efficient cross-linking.

Then, a second experiment was performed to determine the effect of temperature on the cross-linking reaction. The increase of elastic modulus G' overtime has been investigated for the first composition as described above and for a third composition, identical to the first composition. The first composition was incubated at 20 °C without stirring (Example in Fig. 12) whereas the third composition was incubated at 40 °C (Comparative Example 2 in Fig. 12).

As visible in Fig. 12, elastic modulus G' for the Comparative Example 2 does not increases overtime, indicating no or very limited cross-linking. In contrast, elastic modulus G' for the Example (i.e. the present hydrogel 2% w/v) increases strongly overtime, indicating an efficient cross-linking. The optimal temperature for an efficient cross-linking is thus 15 to 25 °C, preferably around 20 °C.

Then, the effect of pH on the cross-linking reaction has been investigated in view of the elastic modulus G' obtained by the obtained hydrogel (2% w/v) at 20 °C. The imines moieties are formed when the primary amines moieties react with aldehyde or ketone moieties under appropriate conditions. Imine formation usually requires an acid catalyst. The acid catalyst may allow elimination of water. On the other hand, the mechanism involves unprotonated amines for a nucleophile attack to the carbonyl function.

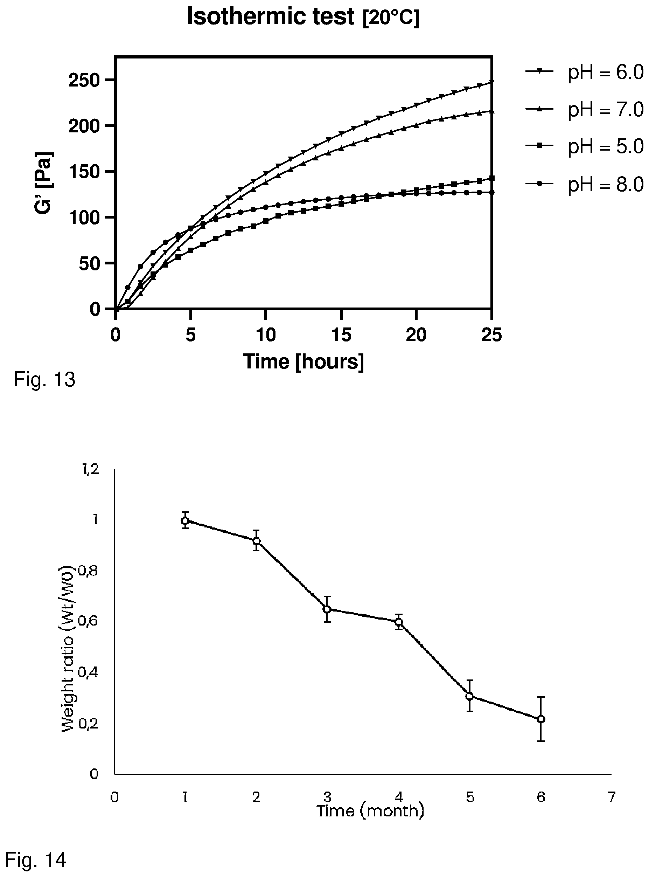

Fig. 13 shows the elastic modulus (G') of 2% w/v hydrogels obtained from cross-linking reactions in different pH buffers and in different reaction time (0 to 25 hours). The elastic modulus was measured as disclosed above. Higher elastic modulus are obtained for pH 6.0 and 7.0 after only 10 hours of reactions. A cross-linking reaction in phosphate buffer at pH = 6.0 lead to a hydrogel with a higher elastic modulus compared to pH = 7.0 at a given reaction time, supporting the fact that mild acidic conditions are favourable for the imine formation.

For a hydrogel obtained at pH = 8, a strong increase is shown in the beginning (< 5 h), but the elastic modulus G' is then flattened off, probably due to alkaline degradation of the hydrogel. For a hydrogel obtained at pH = 5, the cross-linking reaction has a limited rate and the elastic modulus, although increasing with the reaction time, cannot match that obtained by hydrogel obtained at pH = 6 or 7 for a given reaction time above 5 hours. In view of the desired elastic modulus G', an optimal pH for the cross-linking reaction may be 6.0 to 7.5. A cross-linking pH of 7.4 allows obtaining the gel in physiological conditions with a high cross-linking rate, thus avoiding an additional step of changing the hydrogel pH.

### In Vitro Toxicity

An in vitro cytotoxicity was performed according to the method of ISO 10993-5:2009 for di-aldehyde nanocrystalline cellulose, nanocrystalline cellulose, and the present hydrogel at 1.0 and 2.0 (w/v)% as shown in Table 1. This demonstrates that the present hydrogel has no cytotoxic effects and may be safely injected, in contrast with di-aldehyde nanocrystalline cellulose.

**[Table 1]**

| **Sample** | **Cell viability reduction (%)** | **Result** |
|---|---|---|
| **Nanocrystalline Cellulose** | 12 ± 0.76 | Not Cytotoxic |
| **di-aldehyde Nanocrystalline Cellulose** | 88 ± 0.76 | Cytotoxic |
| **Hydrogel 1.0%** | 6 ± 0.76 | Not Cytotoxic |
| **Hydrogel 2.0%** | 12 ± 0.76 | Not Cytotoxic |

### Miscibility

Body joints are filled with synovial fluid, and miscibility is a key criterion to evaluate the ability of a hydrogel to treat a joint having OA. Synovial fluid is primarily composed of water, hyaluronan, lubricin, proteinase, collagenases, and prostaglandins and has a pH around 7.5.

Injecting a non-miscible hydrogel into an impaired joint to be treated may provide no or limited benefit in terms of pain and mobility but may trigger a strong inflammatory response, thus degrading the treated joint. Consequently, the miscibility of the present hydrogel has been evaluated in physiological conditions (i.e. PBS buffer, pH = 7.4 at a temperature of 37 °C) and compared to the miscibility of comparative hydrogels described below. The result of this evaluation is visible in Table 2.

**Table 2**

| **Sample** | **Miscibility** |
|---|---|
| Hydrogel 1% w/v | Stable colloid |
| Hydrogel 2% w/v | Stable colloid |
| Hydrogel 4% w/v | Stable colloid |
| Comparative Example 3 | Not Applicable |
| Comparative Example 4 | Not miscible |
| Comparative Example 5 | Not miscible |

The present hydrogels are fully miscible in physiological conditions and form stable colloids in concentration of 1% to 4% w/v. The same miscibility results have been observed in horse synovial fluid (see the above rheological and viscoelastic experiments). Without being bond by any theory, it is believed that the unique chemical structure of the hydrogel allows obtaining a stable colloid in PBS buffer and in synovial fluid, thus obtaining a single phase with favourable viscoelastic and rheological properties.

### Comparative Examples

Comparative Example 3 is obtained by replacing gelatine by collagen in the above method of preparing a hydrogel. The conditions for the cross-linking reaction between collagen and oxidized nanocrystalline cellulose were a temperature of 20 °C without stirring for 10 hours. No cross-linking reaction was performed in the above conditions and no hydrogel was obtained.

Comparative Example 4 relates to a hydrogel prepared according to Example 3 of CN110760103. The hydrogel is prepared by mixing in a container oxidized nanocrystalline cellulose at a concentration of 12 mg/ml and pH = 8, with an identical weight of a pre-polymerized solution of collagen at a concentration of 12 mg/ml and a pH = 5. A thick hydrogel is obtained after a slow mixing at room temperature during half a minute. However, the hydrogel of Comparative Example 4 is not miscible in a PBS buffer at pH = 7.4 or in synovial fluid and two different phases are obtained. Consequently, no rheological measurement as described above can be performed.

In vivo evaluation of the hydrogel of Comparative Example 4 has been performed. A 2% w/v hydrogel according to Comparative Example 4 has been injected subcutaneously to a mouse, and no inflammatory response was observed. Then, the same 2% hydrogel has been injected in three fetlock equine joints with OA and a strong inflammatory response was observed. It is thus apparent that the hydrogel of Comparative Example 4 cannot relieve the symptoms of a joint having OA.

Comparative Example 5 relates to preparing a hydrogel according to the present method and replacing oxidized nanocrystalline cellulose by oxidized carboxymethyl cellulose. In the above cross-linking reaction conditions, a hydrogel is obtained. However, this hydrogel is not miscible in PBS buffer at pH = 7.4 and is not miscible in synovial fluid. Consequently, the hydrogel of Comparative Example 5 does not allow relieving the symptoms of an impaired joint.

Comparative Example 6 relates to performing a cross-linking reaction between oxidized nanocrystalline cellulose and gelatine according to the present method. However, the crosslinking conditions were changed to 37 °C in PBS buffer at pH = 7.4. No crosslinking was obtained under these conditions and no hydrogel was formed. Without being bound by any theory, it is believed that the reaction rate of beta-elimination is greater to the reaction rate of Schiff-base formation, thus preventing an efficient cross-linking reaction.

### In Vitro Stability

In vitro stability of a 2% w/v hydrogel was investigated over 6 months under physiological conditions. 6 samples of hydrogel 2% w/v were prepared and kept at 37 °C and pH = 7.4 under gentle shaking. To determine the weight of hydrogel of each sample, the sample was freeze-dried to remove the liquid phase and the weight (wt) of the gel fraction was obtained from a precision scale. After 2 months, the remaining weight is still higher than 90% of the original weight (wo). After 6 months, around 20% of the original weight (wo) is still present.

This preliminary stability experiment thus demonstrates a good stability of the hydrogel in physiological conditions, thus supporting a long-lasting relief of OA symptoms in vivo.

### Posology

Small horse joints may be treated with 2 ml of 2% w/v hydrogel, while larger horse joints may be treated with 4 ml of 2% w/v hydrogel. For human joint, 1 ml of 2% w/v hydrogel may be used.

Although the present invention has been described and illustrated in detail, it is clearly understood that the same is by way of illustration and example only and is not to be taken by way of limitations, the scope of the present invention being limited only by the terms of the appended claims.

## Claims

1. An injectable hydrogel including:
• oxidized nanocrystalline cellulose,
• gelatine peptide chains,
wherein the oxidized nanocrystalline cellulose and the gelatine peptide chains are cross-linked with a cross-linking degree of 25 to 60%, and
wherein the injectable hydrogel has a concentration of 1% to 4% w/v.

2. The injectable hydrogel of the previous claim, wherein the oxidized nanocrystalline cellulose and the gelatine peptide chains are cross-linked by imine moieties.

3. The injectable hydrogel of anyone of the previous claims, wherein the gelatine peptide chains have a concentration of 1.0 to 2.5 weight/volume % before cross-linking and/or the oxidized nanocrystalline cellulose has a concentration of 0.1 to 0.5 weight/volume % before cross-linking.

4. The injectable hydrogel of anyone of the previous claims, wherein the injectable hydrogel includes 0.5 to 2.5 weight/volume % of cross-linked oxidized nanocrystalline cellulose and gelatine peptide chains.

5. The injectable hydrogel of anyone of the previous claims, wherein the injectable hydrogel has an elastic modulus of 10 to 220 Pa from 0.1 to 10.0 Hz.

6. The injectable hydrogel of anyone of the previous claims, wherein the injectable hydrogel has a loss modulus below 10 Pa from 0.1 to 10.0 Hz.

7. The injectable hydrogel of anyone of the previous claims, wherein the injectable hydrogel has a cross-linking degree of 38 to 49%.

8. A method to prepare an injectable hydrogel according to anyone of the previous claims, the method including the steps of:
- Diluting oxidized nanocrystalline cellulose in apyrogenic water
- Adding gelatine peptide chains to the diluted oxidized nanocrystalline cellulose at room temperature
- Reacting the gelatine peptide chains and the oxidized nanocrystalline cellulose.

9. The method to prepare an injectable hydrogel according to claim 8, wherein the reacting step is performed for 10 to 25 hours, preferably at a temperature of 15 to 25 °C.

10. The method to prepare an injectable hydrogel according to claim 8 or claim 9, wherein the reacting step is performed at a pH of 6 to 7.5.

11. The method to prepare an injectable hydrogel according to any one of claims 8 to 10, further including a step of diluting the gelatine peptide chains in water at 37 °C and the cooling down of the diluted gelatine peptide chains at room temperature.

12. Injectable hydrogel according to anyone of claims 1 to 7 for use to cure or relieve the symptoms of osteoarthritis.

13. A syringe containing a hydrogel according to anyone of claims 1 to 7.

## Patentansprüche

1. Injizierbares Hydrogel, das Folgendes beinhaltet:
• oxidierte nanokristalline Cellulose,
• Gelatinepeptidketten,
wobei die oxidierte nanokristalline Cellulose und die Gelatinepeptidketten mit einem Vernetzungsgrad von 25 bis 60 % vernetzt sind, und
wobei das injizierbare Hydrogel eine Konzentration von 1 % bis 4 % W/V aufweist.

2. Injizierbares Hydrogel nach dem vorhergehenden Anspruch, wobei die oxidierte nanokristalline Cellulose und die Gelatinepeptidketten durch Imineinheiten vernetzt sind.

3. Injizierbares Hydrogel nach einem der vorhergehenden Ansprüche, wobei die Gelatinepeptidketten vor der Vernetzung eine Konzentration von 1,0 bis 2,5 Gew./Vol.-% aufweisen und/oder die oxidierte nanokristalline Cellulose vor der Vernetzung eine Konzentration von 0,1 bis 0,5 Gew./Vol.-% aufweist.

4. Injizierbares Hydrogel nach einem der vorhergehenden Ansprüche, wobei das injizierbare Hydrogel 0,5 bis 2,5 Gew./Vol.-% vernetzte oxidierte nanokristalline Cellulose und Gelatinepeptidketten beinhaltet.

5. Injizierbares Hydrogel nach einem der vorhergehenden Ansprüche, wobei das injizierbare Hydrogel einen Elastizitätsmodul von 10 bis 220 Pa von 0,1 bis 10,0 Hz aufweist.

6. Injizierbares Hydrogel nach einem der vorhergehenden Ansprüche, wobei das injizierbare Hydrogel einen Verlustmodul unter 10 Pa von 0,1 bis 10,0 Hz aufweist.

7. Injizierbares Hydrogel nach einem der vorhergehenden Ansprüche, wobei das injizierbare Hydrogel einen Vernetzungsgrad von 38 bis 49 % aufweist.

8. Verfahren zur Herstellung eines injizierbaren Hydrogels nach einem der vorhergehenden Ansprüche, wobei das Verfahren die folgenden Schritte umfasst:
- Verdünnen von oxidierter nanokristalliner Cellulose in apyrogenem Wasser
- Zugeben von Gelatinepeptidketten zu der verdünnten oxidierten nanokristallinen Cellulose bei Raumtemperatur
- Umsetzen der Gelatinepeptidketten und der oxidierten nanokristallinen Cellulose.

9. Verfahren zur Herstellung eines injizierbaren Hydrogels nach Anspruch 8, wobei der Schritt des Umsetzens während 10 bis 25 Stunden, vorzugsweise bei einer Temperatur von 15 bis 25 °C, durchgeführt wird.

10. Verfahren zur Herstellung eines injizierbaren Hydrogels nach Anspruch 8 oder Anspruch 9, wobei der Schritt des Umsetzens bei einem pH-Wert von 6 bis 7,5 durchgeführt wird.

11. Verfahren zur Herstellung eines injizierbaren Hydrogels nach einem der Ansprüche 8 bis 10, ferner umfassend einen Schritt des Verdünnens der Gelatinepeptidketten in Wasser bei 37 °C und des Abkühlens der verdünnten Gelatinepeptidketten bei Raumtemperatur.

12. Injizierbares Hydrogel nach einem der Ansprüche 1 bis 7 zur Verwendung zur Heilung oder Linderung der Symptome von Osteoarthritis.

13. Spritze, enthaltend ein Hydrogel nach einem der Ansprüche 1 bis 7.

## Revendications

1. Hydrogel injectable comprenant :
• une cellulose nanocristalline oxydée,
• des chaînes peptidiques de gélatine,
dans lequel la cellulose nanocristalline oxydée et les chaînes peptidiques de gélatine sont réticulées avec un degré de réticulation de 25 à 60 %, et
l'hydrogel injectable ayant une concentration de 1 % à 4 % p/v.

2. Hydrogel injectable selon la revendication précédente, dans lequel la cellulose nanocristalline oxydée et les chaînes peptidiques de gélatine sont réticulées par des groupements imine.

3. Hydrogel injectable selon l'une quelconque des revendications précédentes, dans lequel les chaînes peptidiques de gélatine ont une concentration de 1,0 à 2,5 % en poids/volume avant réticulation et/ou la cellulose nanocristalline oxydée a une concentration de 0,1 à 0,5 % en poids/volume avant réticulation.

4. Hydrogel injectable selon l'une quelconque des revendications précédentes, dans lequel l'hydrogel injectable comprend 0,5 à 2,5 % en poids/volume de cellulose nanocristalline oxydée réticulée et de chaînes peptidiques de gélatine réticulées.

5. Hydrogel injectable selon l'une quelconque des revendications précédentes, dans lequel l'hydrogel injectable a un module d'élasticité de 10 à 220 Pa de 0,1 à 10,0 Hz.

6. Hydrogel injectable selon l'une quelconque des revendications précédentes, dans lequel l'hydrogel injectable a un module de perte inférieur à 10 Pa de 0,1 à 10,0 Hz.

7. Hydrogel injectable selon l'une quelconque des revendications précédentes, dans lequel l'hydrogel injectable a un degré de réticulation de 38 à 49 %.

8. Procédé de préparation d'un hydrogel injectable selon l'une quelconque des revendications précédentes, le procédé comprenant les étapes de :
- dilution d'une cellulose nanocristalline oxydée dans de l'eau apyrogène
- ajout de chaînes peptidiques de gélatine à la cellulose nanocristalline oxydée diluée à température ambiante
- mise en réaction des chaînes peptidiques de gélatine et de la cellulose nanocristalline oxydée.

9. Procédé de préparation d'un hydrogel injectable selon la revendication 8, dans lequel l'étape de mise en réaction est réalisée pendant 10 à 25 heures, de préférence à une température de 15 à 25 °C.

10. Procédé de préparation d'un hydrogel injectable selon la revendication 8 ou la revendication 9, dans lequel l'étape de mise en réaction est réalisée à un pH de 6 à 7,5.

11. Procédé de préparation d'un hydrogel injectable selon l'une quelconque des revendications 8 à 10, comprenant en outre une étape de dilution des chaînes peptidiques de gélatine dans de l'eau à 37 °C et le refroidissement des chaînes peptidiques de gélatine diluées à température ambiante.

12. Hydrogel injectable selon l'une quelconque des revendications 1 à 7, destiné à être utilisé pour guérir ou soulager les symptômes de l'arthrose.

13. Seringue contenant un hydrogel selon l'une quelconque des revendications 1 à 7.
